# EUROPEAN PATENT SPECIFICATION

(11) **EP 1 385 523 B1**
(45) Date of publication and mention of the grant of the patent: **06.08.2008**
(21) Application number: 02769372.0
(22) Date of filing: 08.05.2002
(51) Int. Cl.: A61K 31/517, A61K 31/522, A61P 25/28

(54) **ADENOSINE A1 RECEPTOR ANTAGONISTS FOR TREATING HYPOXIA-INDUCED LEARNING AND MEMORY IMPAIRMENT**
ADENOSIN-A1-REZEPTORANTAGONISTEN ZUR BEHANDLUNG VON HYPOXIEBEDINGTEN LERN- UND GEDÄCHTNISSTÖRUNGEN
ANTAGONISTES DU RECEPTEUR DE L'ADENOSINE A1 PERMETTANT DE TRAITER DES TROUBLES DE LA MEMOIRE ET DE L'APPRENTISSAGE INDUITS PAR UNE HYPOXIE

(30) Priority: 08.05.2001 US 289137 P
(43) Date of publication of application: 04.02.2004
(73) Proprietor: Blanchette Rockefeller Neurosciences Institute, Rockville, MD 20850 (US)
(72) Inventor: SUN, Miao-Kun, Bethesda, MD 20878 (US); ALKON, Daniel, L., Bethesda, MD 20817 (US)
(74) Representative: Kador & Partner
(86) International application number: PCT/US2002/014378
(87) International publication number: WO 2002/089736

(56) References cited:
- US-B1- 6 187 780
- MIAO-KUN SUN ET ALL.: "Pharmacological protection of synaptic function, spatial learning, and memory from transient hypoxia in rats." THE JOURNAL OF PHARMACOLOGY AND EXPERIMENTAL THERAPEUTICS, vol. 300, no. 2, February 2002 (2002-02), pages 408-416, XP002308471
- CANHAO, P ET ALL.: "1,3-dipropyl-8-cyclopentylxanthine attenuates the NMDA response to hypoxia in the rat hippocampus" BRAIN RESEARCH, vol. 661, no. 1/2, 1994, pages 265-273, XP002308472
- BONA E ET ALL.: "Neonatal cerebral hypoxia-ischemia: the effect of adenosine receptor anatgonists" NEUROPHARMACOLOGY, vol. 36, no. 9, 1997, pages 1327-1338, XP002308473
- LONGO, R ET ALL.: "Opposite modulation of 4-aminopyridine and hypoxic hyperexicatability by A1 and A2 adenosine receptor ligands in rat hipocampal slices" NEUROSCIENCE LETTERS, vol. 200, no. 1, 1995, pages 21-24, XP002308474
- DAG K.J.E. VON LUBITZ ET ALL.: "Effects of the chronic administration of adenosine A1 receptor agonist and antagonist on spatial learning and memory" EUROPEAN JOURNAL OF PHARMACOLOGY, vol. 249, 1993, pages 271-280, XP002308475
- SILVIA R. KOPF ET ALL.: "Adenosine and memory storage: effects of A1 and A2 receptor antagonists" PSYCHOPHARMACOLOGY, vol. 146, 1999, pages 214-219, XP002308476
- TABATA ET AL.: 'Ameliorative effects of paeoniflorin, a major constituent of peony root, on adenosine A1 receptor-mediated impairment of passive avoidance performance and long term potentiation in the hippocampus' BIOL. PHARM. BULL. vol. 24, no. 5, 2001, pages 496 - 500, XP002959595
- DATABASE BIOSIS [Online] OCHIISHI ET AL.: 'High level of adenosine A1 receptor-like immunoreactivity in the CA2/CA3a region of the adult rat hippocampus', XP002959596 Database accession no. (PREV199900424583) & NEUROSCIENCE vol. 93, no. 3, 04 August 1999, pages 955 - 967

## Description

This application claims the benefit of provisional application USSN 60/289,137, filed May 8, 2001, incorporated herein by reference.

### FIELD OF THE INVENTION

The invention relates to a therapeutic formulation for treating or preventing memory loss produced by reversible transient hypoxia-induced and associated synaptic dysfunction comprising administering an adenosine A1 receptor antagonist.

### BACKGROUND

Hypoxia and ischemic stroke remain one of the most devastating threats to humans. Memory impairment is common after cerebral hypoxia/ischemia, bypass surgery, or heart ttack¹. Although all mammalian cells can sense and will respond to hypoxia^{2,3}, hippocampal CA1 pyramidal cells are among, if not the, most sensitive. Hypoxic/ischemic consequences consist mainly of three forms: functional disruptions, cellular injury and delayed cell loss through apoptosis⁴ or necrosis, depending on the severity of the insult. Each form has distinct pathophysiological characterization and requires different therapeutics.

It is known that a selective adenosine A1 receptor antagonist, DPCPX, mitigates hypoxia-induced accumulation of adenosine during hypoxia. Pearson T. et al., Eur. J. Neurosci. 2000, 12(8):3064-6. Adenosine suppresses synaptic responses in rat hippocampus during hypoxia, and that suppression was reversed by use of an A1 antagonist. Arlinghaus et al., Brain Res. 1996, 724(2):265-8. Adenosine-mediation of anoxia induced synaptic glutamate release in CA1 pyramidal neurons was not affected by DPCX. Katchman et al., Hippocampus 1996, 6(3):213-24. and U.S. Patent 6.166,181. Another antagonist blocked hypoxia-induced depression of synaptic transmission in CA1 neurons. Doolette et al., Brain Res. 1995, 677(1):127-37. These references do not demonstrate or teach any effect of hypoxia on hippocampal theta rhythm, attention, learning, or memory, or an effect of blockade of CA1 adenosine A1 receptors in preventing hypoxia-induced memory loss.

Spatial learning and memory depend on information processing by the hippocampal networks, whose function is extremely sensitive to mild hypoxia and transient ischemia. However, despite intensive research aimed at the development of effective therapeutic interventions, promising therapy is still lacking. The present invention demonstrates that reversible transient hypoxia reduced cholinergic; θ activity and associated synaptic "arrest' in hippocampal CA1, and that these response were preventable by adenosine A₁ receptor antagonism. Brief hypoxic episodes markedly impaired the ability of rats in a Morris water-maze spatial learning and memory. The impairment was prevented by adenosine A₁ receptor antagonism. This protection of synaptic efficacy represents an effective therapeutic strategy to eliminate functional interruption due to brief hypoxic episodes. Moreover, the present invention provides a molecule with high log P and that readily enters the central nervous system or one that may be transferred or "locked" into the brain through the use of a pro-drug technique.

### SUMMARY OF THE INVENTION

The invention relates to a therapeutic formulation comprising a pharmaceutically acceptable composition comprising an adenosine A-1 antagonist, the composition delivering the antagonist across the blood brain barrier, the composition not causing any unwanted side effects in concentrations effective to block learning and/or memory-loss related lesions caused by hypoxia. The invention also relates to an article of manufacture consisting essentially of a pharmaceutically acceptable composition, packaged together with instructions indicating use in connection with mitigating hypoxia-induced lesions. The adenosine A1 receptor antagonist can be 8-cyclopentyl-1,3-dipropylxanthine(CPDPX). The formulation can comprise a combination of adenosine A-1 antagonist with an agent that reverses cellular injury and/or prevents cell loss.

The invention relates to a method of identifying therapeutic A-1 antagonist compounds useful for treating hypoxia-related memory loss comprising: providing brain tissue under controlled conditions modeling theta activity, placing the tissue under conditions of hypoxia causing synaptic arrest and loss of theta activity, administering a candidate A-1 antagonist compound to the brain tissue under conditions of hypoxia, and determining whether the candidate compound prevents synaptic arrest of the brain tissue and/or maintain theta activity. The brain tissue can comprise CA1 pyramidal cells.

Further objectives and advantages will become apparent from a consideration of the description, drawings, and examples.

### BRIEF DESCRIPTION OF THE DRAWINGS

The invention is better understood by reading the following detailed description with reference to the accompanying figures:

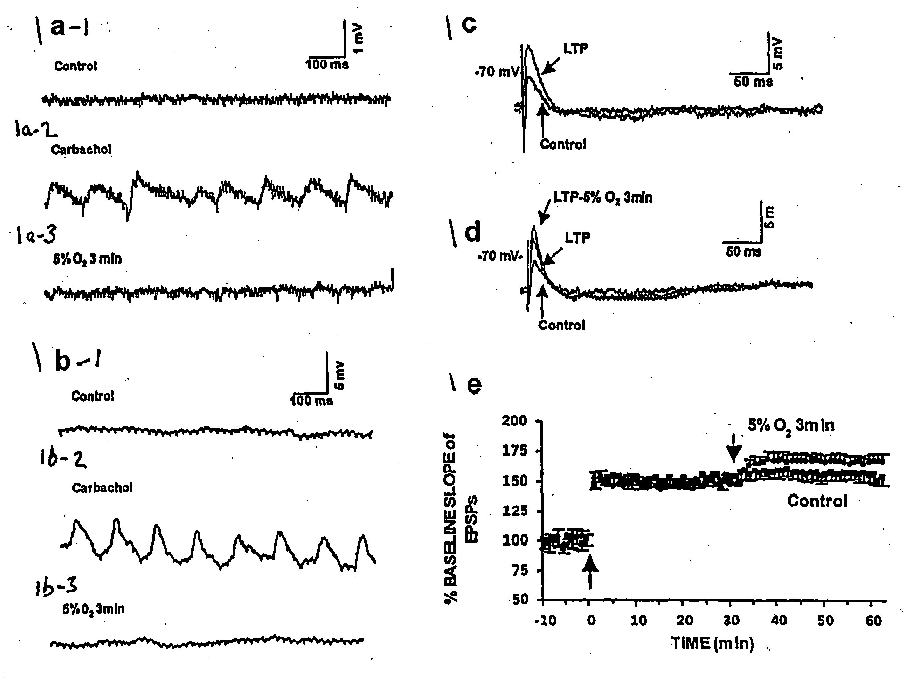

Figures 1a-1, 1a-2, 1a-3, 1b-1, 1b-2, 1b-3, 1c, 1d and 1e show the differential effects of brief hypoxia on cholinergic CA1 θ and long-term potentiation of Sch-CA1 EPSPs. Examples of recorded field potentials: pre-carbachol control (Fig. 1a-1), during carbachol (50uM, 30 min; Fig. 1a-2) and 10 min after brief hypoxia (5% O₂ 3min; Fig. 1a-3). Membrane potential traces of recorded CA1 pyramidal cells: pre-carbachol (control; Fig. 1b-1), during carbachol application (50 µM, 30 min; Fig. 1b-2), and 10 min after brief hypoxia (5% O₂ 3min; Fig. 1b-3). The membrane was maintained at pre-carbachol level by passing negative current (the second trace). Representative Sch-CA1 EPSP traces (Fig. 1c) of post-HFS (LTP, 40 min after HFS) and pre-HFS (Control). Representative Sch-CA1 EPSP traces (Fig. 1d) of pre-HFS (Control), post-HFS (LTP, 29 min after HFS) and immediately after brief hypoxia (5% O₂ 3min). Fig. 1e represents time course of Sch-CA1 EPSPs in response to HFS (at the first arrow) and brief hypoxia (at the second arrow). Data points are mean±S.E. of the mean. EPSPs were evoked 1/min. For clarity, only every other points are shown ■: control; ●: 5% O₂ for 3 minutes.

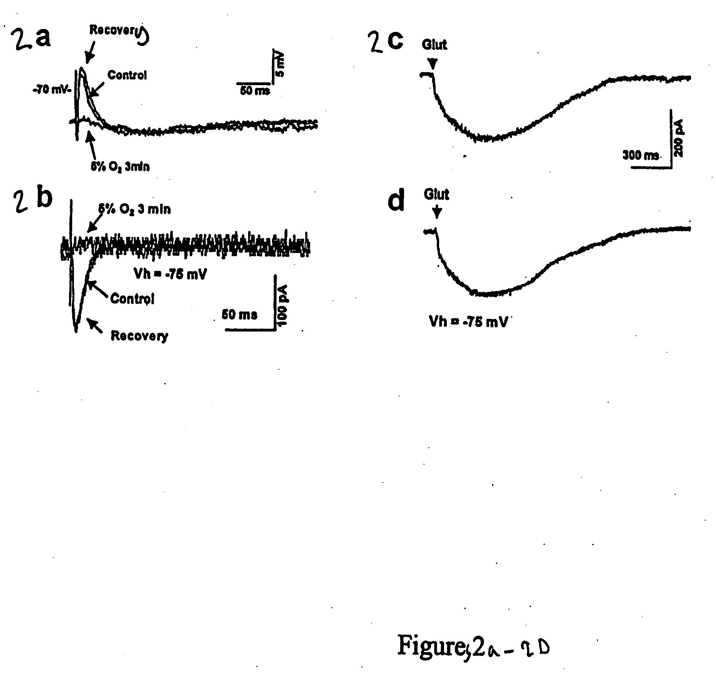

Figures 2a, 2b, 2c and 2d demonstrate the synaptic arrest produced by brief hypoxia without causing obvious cellular loss. Sch-CA1 EPSPs (Fig. 2a) and EPSCs (Fig. 2b) were briefly abolished at the end of brief hypoxia (5% O₂ 3min), as compared with those of the next trace (Recovery) and of pre-hypoxia (Control). Representative traces of membrane response to local application of glutamate (Glut; 20 µl of 10 mM) before (Fig. 2c) and at the end of brief hypoxia (Fig. 2d); with glutamate application (20 µl of 10 mM) about 0.5 s before the end of the 3 min hypoxia so that the peak was about the end of the 3min). Nissl stained coronal sections of the dorsal CA1 field revealed densely packed pyramidal cells with well-defined nuclei in control rats and rats subjected to 8 episodes of brief hypoxia (not shown).

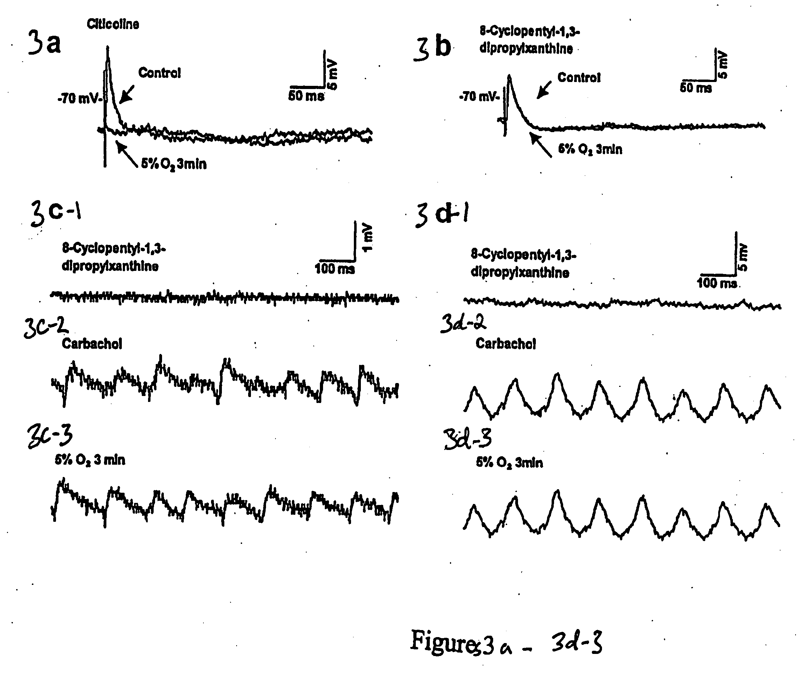

Figures 3a, 3b, 3c-1, 3c-2, 3c-3, 3d-1, 3d-2 and 3d-3 show the effects of adenosine A₁ receptor antagonist on synaptic arrest, CA1 θ, in response to brief hypoxia. In the presence of citicoline (100 µM), synaptic arrest was observed at the end of the 3 min hypoxia (Fig. 3a). In the presence of 8-cyclopentyl-1,3-dipropylxanthine (10 µM), synaptic arrest was abolished (Fig. 3b) and brief hypoxia neither eliminated cholinergic CA1 0 (Fig. 3c-1-3) nor cholinergic intracellular 0 of the CA1 pyramidal cells (Fig. 3d-1-3).

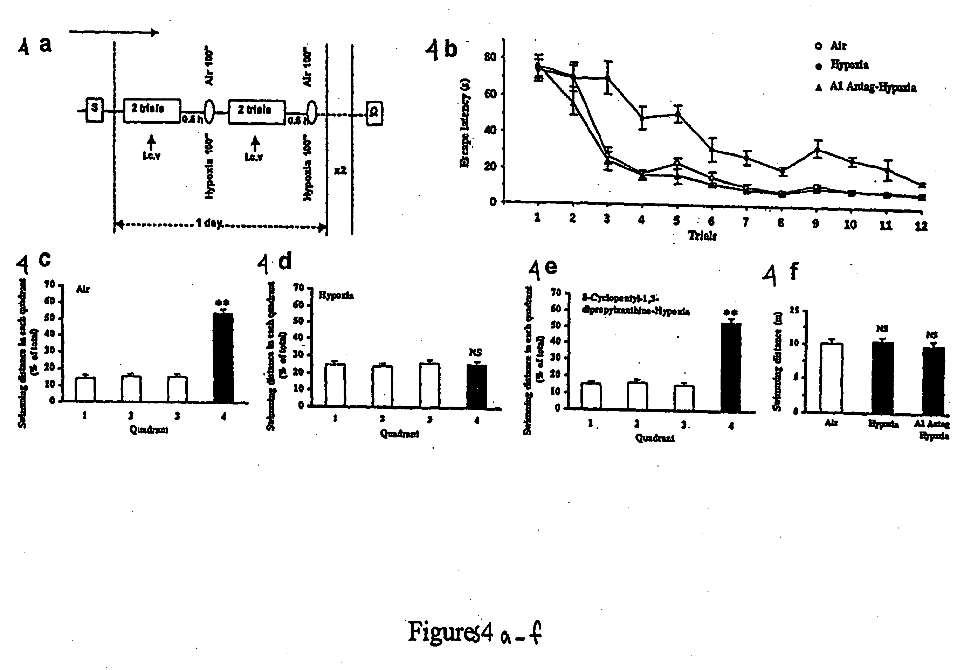

Figures 4a, 4b, 4c, 4d, 4e and 4f demonstrate the effects of brief hypoxia and adenosine A₁ receptor antagonist on rat performance in the hidden platform water maze task. The figure illustrates experimental protocol (Fig. 4a), escape latency (means ± SEM) in water maze training (Fig. 4b) across 12 trials (*F_{11,312}* = 50.14,p<0.0001), and quadrant preference (Fig. 4c, 4d and 4e) conducted at the end of the twelfth training session, and swimming distance (in 1 min; Fig. 4f). Rats were either subjected to air or hypoxia (95% N₂/5% CO₂ for 100 s) about 30 min in a glass jar after the 2nd or 4th trial of the day. Bilateral i.c.v. CPDPX (400 nmoles/site) or vehicle were administered before the 2nd and 4th trials of the day. Quadrant 4 is the target quadrant during training.

### DETAILED DESCRIPTION

In describing preferred embodiments of the present invention, specific terminology is employed for the sake of clarity.

The invention provides therapeutic compositions targeted to lesions induced by hypoxia leading to memory loss. These lesions have biochemical, physiological, and cognitive aspects, all of which are related and may be considered as targets subject to therapy according to the invention. The biochemical target for the methods and formulations for the invention is the adenosine A-1 receptor in neurons associated with memory and learning, in particular those which are affected by hypoxia. The targeted receptors respond to adenosine signals during hypoxia in a cascade causing synaptic arrest and memory Impairment, without cell damage or death.

The physiological aspect of lesions targeted by the invention is the reversible condition of synaptic arrest and reduction of cholinergic theta induction of the CA1 neuronal network. This network includes CA1 pyramidal neurons and others involved in generating stable theta activity and subject to synaptic arrest during hypoxia. During hypoxia, EPSPs and EPSCs are eliminated, the CA1 neuronal network becomes disconnected, and theta activity is reversibly lost until oxygen is applied again.

The cognitive/behavioral lesions subject to therapy may be characterized generally as attention impairment, learning impairment, memory impairment, including amnesia, the loss of memory, memory retention, and learning including spatial learning. The impairment may be sudden as in transient hypoxia, or long term and gradual, or both, as may occur with repeated incidents of transient hypoxia. Such chronic or repeated incidents may lead to other lesions as well.

Subjects in need of the therapy are those exposed to hypoxia from any source. Generally, subjects for therapy are those at risk for hypoxia, including older people, people with chronic obstructive lung disease, people entering surgery, those at risk of stroke, and others having diseases predisposing them to hypoxia. Hypoxia induces many lesions in subjects, including cell death and a wide variety of synaptic dysfunction. Subjects in need of the therapy are those facing hypoxia-induced theta rhythm abnormality and memory loss.

Only that synaptic dysfunction associated with memory loss is subject to therapy here, and other hypoxia effects are not subject to treatment according to the invention. For example, hypoxia interferes with long term potentiation (LTP) but treatment with an adenosine A1 receptors antagonist does not mitigate that interference. It was not predictable that A1 antagonists would work on CA1 neurons and/or others involved in generating theta activity and supporting memory retention.

The hypoxia subject to therapy is mild. i.e. causing reversible effects, but sufficient to interrupt theta activity and/or intracellular theta, without causing cell loss. The hypoxia subject to therapy causes low brain oxygen levels but not substantial immediate cell death. Causes of the hypoxia inducing the lesions targeted by the invention include traumatic events, transient ischemic attack, surgery-related hypoxia, acute aud/or chronic obstructive lung disease, central nervous system infections such as meningitis encephalitis and/or other traumatic injury of the central nervous system.

Repeated hypoxic episodes of the type subject to therapy may be associated with and/or precede neurodegeneration over time. Such disorders include Alzheimer's Disease, Parkinson's, Pugilistia or dementia.

The invention reduces or eliminates the lesions induced by hypoxia. The inventive therapy blocks adenosine A1 receptors on the targeted neurons. This blockade protects and enhances synaptic efficacy and eliminates interruption of, or reduces synaptic dysfunction referred to here as synaptic arrest leading to loss of stable theta rhythm. The methods and compositions provide therapy for a condition of impaired memory in a subject exposed to hypoxia, treat or prevent memory loss, blocking or mitigating the extent of the cognitive impairment.

Therapy means administering an adenosine A1 receptor antagonist to neurons involved in generating theta rhythm, in an amount effective to prevent synaptic arrest induced by hypoxia. The antagonist most be administered in a dose and manner effective to cross the blood brain barrier to provide a blockade effect at the time it is needed, i.e. during hypoxia.

Aspect of the invention relates to reducing the effects of reversible transient hypoxia and associated synaptic dysfunction. The hypoxia effects according to the invention may involve reductions of about 50% to about 95%, e.g. about 75%, about 80%, or about 90%, of synaptic transmission, theta activity and/or intracellular theta activity. Using a selective adenosine A₁ receptor antagonist according to the invention may mitigate the effects of hypoxia by restoring about 75% to about 100%, e.g about 80, 90, 95 or 99%, of pre-hypoxia levels for synaptic transmission and/or theta and intracellular theta activity.

The formulations of the invention are pharmaceutically acceptable compositions comprising adenosine A-1 antagonists. Particularly useful in the invention are those antagonists which can cross the blood brain barrier and do not cause any unwanted side effects in concentrations effective to block the memory-loss-Mated lesions caused by hypoxia. According to the invention, a commercial product is provided consisting essentially of such a pharmaceutically acceptable composition packaged together with instructions indicating use in confection with mitigating hypoxia-induced lesions.

The invention provides a method for relieving hypoxia-induced memory loss in a subject exposed to hypoxia, comprising administering to the subject an adenosine A1 receptor antagonist in an amount effective to prevent synaptic arrest leading to loss of theta rhythm. The invention provides a method for preventing hypoxia induced, reversible synaptic arrest in a subject by blocking adenosine **A1** receptors in the brain of the subject.

The method of treating a neurodegenerative disorder comprising administering an effective amount of an adenosine A1 receptor antagonist (in combination with an effective amount of an agent that reverses cellular injury and prevent cell loss). The invention relates to a pharmaceutical compositions comprising an adenosine A1 receptor antagonist and a pharmaceutically acceptable carrier, the composition delivering the antagonist across the blood brain barrier. In another embodiment, the invention relates to a method of maintaining theta activity during hypoxia, comprising administering an adenosine A1 antagonist to the brain in an elective amount. Another aspect relates to a method of preventing or reversing synaptic arrest of CA1 neurons due to hypoxia in the absence of cellular injury.

Brief hypoxia impairs functioning of CA1 neuronal synaptic transmission, long-term potentiation (LTP) of glutamatergic EPSPs, and cholinergic θ, a memory-related neuronal activity synchronization that depends on a temporal heterosynaptic interaction⁵. In addition, brief hypoxia blocks synaptic transmission⁶ of glutamatergic inputs, GABAergic inputs and cholinergic inputs, causing disconnection, or synaptic 'arrest', of the CA1 neuronal network. Many of these inputs and their interaction play an essential role in enhancing synaptic efficacy in learning and memory.

In experiments conducted by the inventors, brief hypoxia eliminated EPSPs and EPSCs temporarily. The synaptic 'arrest' immediately disappeared when reoxygenation was initiated and was not produced postsynaptically. The hypoxic synaptic 'arrest' and reduction in cholinergic 0 induction were prevented by blocking the adenosine A₁ receptors. Application of citicoline, a neuroprotective substance, on the other hand, is ineffective suggesting that cellular injury is not involved.

In the presence of 8-pentyl-1,3-dipropylxanthine (CPDPX), a selective adenosine A₁ receptor antagonist, synaptic transmission remained intact at the end of the hypoxia. Neither the θ activity nor intracellular θ were affected by the brief hypoxia.

The inventors have demonstrated that a selective adenosine A₁ receptor antagonist, such as 8-cyclopentyl-1,3-dipropylxanthine (CPDPX), can be utilized to eliminate the functional impairment associated with transient hypoxia-induced memory loss and associated synaptic dysfunction. This can be achieved by relieving the network from heterosynaptic 'arrest' by blocking the adenosine A₁ receptors.

The present invention further comprises combining the selective adenosine A₁ receptor antagonist with agents that reverse cellular injury and prevent cell loss. In addition, the antagonists might also be valuable in therapy against severe hypoxia/ischemia-induced memory loss.

The present invention is employed to treat disorders of impaired neurotransmission by administering a selective adenosine A1 receptor antagonist in effective amounts. Such disorders may include traumatic brain or spinal cord injury or a neurologic or neuromuscular disease such as myasthenia gravis, multiple sclerosis, Alzheimer's disease, or spinal disorders. In addition, the present invention provides a pharmaceutical composition and a pharmaceutically acceptable carrier.

General methods for blocking adenosine A1 receptors are well known. Many adenosine A-1 antagonists are known and persons having ordinary skill in the art may identify more by conventional screening methods. See U.S. Patent No. 6,166,181 to Jacobson et al., and Joel Linden, *Structure and Function of A1 adenosine receptors,* The FASEB Journal, V5:2668-2676 (September 1991), incorporated herein by reference in their entirety. Particular examples of adenosine A-1 antagonists are 8-cyclopentyl-1,3-dipropylxanthine(CPDPX), 1,3-diethyl-8-phenylxanthine (DPX), 8-(p-sulfophenyl)theophylline, BWA-844U, XAC, CGS-15943, BWA-1433U, CP-68,247, XCC, 8-PT, DPSPX and CP-66,713.

Therapeutic methods of administering a pharmaceutical composition to the brain of a subject exposed to hypoxia. The chemical compositions useful in the present invention can be "converted" into pharmaceutical compositions by dissolution in, and/or the addition of, appropriate, pharmaceutically acceptable carriers or diluents. Thus, the compositions may be formulated into solid, semi-solid, liquid, or gaseous preparations, such as tablets, capsules, powders, granules, ointments, solutions, suppositories, injectables, inhalants, and aerosols, using conventional means. Known methods are used to prevent release or absorption of the active ingredient or agent until it reaches the target cells or organ or to ensure time-release of the agent. A pharmaceutically acceptable form is one that does not inactivate or denature the active agent. In pharmaceutical dosage forms useful herein, the present compositions may be used alone or in appropriate association or combination with other pharmaceutically active compounds.

Accordingly, the pharmaceutical compositions of the present invention can be administered to any of a number of sites of a subject and thereby delivered via any of a number of routes to achieve the desired effect. Local or systemic delivery is accomplished by administering the pharmaceutical composition via injection, infusion or sintillation into a body part or body cavity, or by ingestion, inhalation, or insufflation of an aerosol. Preferred routes of administration include parenteral administration, which includes intramuscular, intracranial, intravenous, intraperitoneal, subcutaneous intradermal or topical routes.

The present compositions can be provided in unit dosage form, wherein each dosage unit, e.g., a teaspoon, a tablet, a fixed volume of injectable solution, or a suppository, contains a predetermined amount of the composition, alone or in appropriate combination with other pharmaceutically active agents. The term "unit dosage form" refers to physically discrete units suitable for a human or animal subject, each unit containing, as stated above, a predetermined quantity of the present pharmaceutical composition or combination in an amount sufficient to produce the desired effect. Any pharmaceutically acceptable diluent or carrier may be used in a dosage unit, e.g., a liquid carrier such as a saline solution, a buffer solution, or other physiologically acceptable aqueous solution), or a vehicle. The specifications for the novel unit dosage forms of the present invention depend on the particular effect to be achieved and the particular pharmacodynamic properties of the pharmaceutical composition in the particular host.

An "effective amount" of a composition is an amount that produces the desired effect in a host, which effect can be monitored, using any end-point known to those skilled in the art. The methods described herein are not intended to be all-inclusive, and further methods known to those skilled in the art may be used in their place.

Brain tissue according to the invention may be in situ (in a subject's brain) or in vitro (e.g. a brain tissue biopsy or slice) under controlled conditions modeling theta activity. Furthermore, the amount of each active agent exemplified herein is intended to provide general guidance of the range of each component which may be utilized by the practitioner upon optimizing these methods for practice either in vitro or in vivo. Moreover, exemplified dose ranges do not preclude use of higher or lower doses as might be warranted in a particular application. For example, the actual dose and schedule may vary depending on (a) whether a composition is administered in combination with other pharmaceutical compositions, or (b) inter-individual differences in pharmacokinetics, drug disposition, and metabolism. Similarly, amounts may vary for in vitro applications. One skilled in the art can easily make any necessary adjustments in accordance with the necessities of the particular situation.

### EXAMPLES

### Methods

Brain slices and electrophysiology. Male Sprague-Dawley rats (150-200 gm) were anesthetized with pentobarbital and the brains were removed and cooled rapidly in aCSF solution, bubbled continuously with 95% O₂ and 5% CO₂. Hippocampi were sliced (400 µM) and placed in oxygenated aCSF (NaCI, 124 mM; KCl 3; MgSO₄ 1.3; CaCl₂ 2.4; NaHCO₃ 26; NaH₂PO₄ 1.25; and glucose 10). The CA1 pyramidal cells were recorded at 30-31 °C with sharp electrodes (tip resistance: 60-120 MΩ). Study was performed on CA1 neurons with stable resting membrane potential more negative than -70 mV. Unless otherwise mentioned, test stimuli were applied at frequency of 1 per minute (0.017 Hz). Signals were amplified with AxoClamo-2B amplifier, digitized and stored using DigiData 1200 with the P-Clamp data collection and analysis software (Axon Instruments, Inc.).

Hypoxia. Episodes of hypoxia were induced by replacing the oxygen supply with 95% N₂ /5% O₂ / 5% CO₂ for 3 min or 95% N₂ /5% CO₂ for 100 s. The neuronal responses to either were found to be identical in preliminary experiments. The hypoxia is milder than those used by others to produce an irreversible impairment of synaptic transmission²⁵.

Histology. At the end of behavioral testing, the rats were perfused transcardially under deep terminal pentobarbital anesthesia with 400 ml of 10% formaldehyde. Perfused brains were embedded in wax. Coronal 7-µm sections were cut by a rotary microtome and serial sections through the hippocampal formation were mounted on slides, and processed for Nissl staining.

Spatial maze tasks. Male adult Wistar rats (200-250 gm) were anesthetized with sodium pentobarbital (60 mg/kg, i.p) and placed in a stereotactic apparatus (Kopf Instruments, Tujunga, CA). Two stainless steel guide cannulas were placed with the tips positioned at the coordinates (anterior-posterior, 0.5 mm; lateral, 1.5 mm; horizontal, 3.2 mm), under aseptic conditions. A 7-day recovery period was allowed before any further experimentation. All rats were randomly assigned to different groups (10 each) and swam for 2 min in a 1.5 m (diameter) x 0.6 m (depth) pool (22 ± 1 °C). On the following day, rats were trained in a 2 trial per day task for 4 consecutive days. Each training trial lasted for up to 2 min, during which rats learned to escape from water by finding a hidden platform that was placed at a fixed location and submerged about 1 cm below the water surface. The navigation of the rats was tracked by a video-camera. The quadrant test (1 min) was performed after removing the platform, 24 hrs after the last training trial.

### Results

Effects of brief hypoxia on functions of CA1 neurons were monitored on synaptic transmission, long-term potentiation (LTP) of glutamatergic EPSPs, and cholinergic θ, a memory-related neuronal activity synchronization that depends on a temporal heterosynaptic interaction. Bath application of carbachol (50µM, 20 min), a cholinergic receptor agonist, to hippocampal slices mimicked septal activation and diffuse acetylcholine transmission and induced CA1 θ field potential (Fig. 1a; peak amplitude: 0.73±0.02 mV, n=10, *p*<0.05, at 7.4±0.7 Hz from background noise). The θ is sensitive to atropine blockade and lasted for more than 3h⁷. The θ oscillation of membrane potential (7.5±1.0 mV; n=18;*p*<0.05) was also observed in CA1 pyramidal cells (intracellular θ; Fig. 1b). Brief hypoxia, induced about 30 min after θ induction, greatly reduced θ activity by 87.4% (±5.2%; n=8, *p*<0.05; Fig. 1a) and intracellular θ by a similar extent (by 88.2±4.9%, n=9, *p*<0.05; Fig. 1b). LTP of responses to schaffer collateral (Sch) glutamatergic inputs (Fig. 1c,e), however, was not reduced, but enhanced, by the hypoxia (Fig. 1d, e), consistent with reported hypoxic LTP⁸ and the observation that LTP expression is not vulnerable to transient hypoxia a few minutes after hypoxia. The synaptic transmission was briefly blocked only at the very end of the 3 min of hypoxia⁹.

The period of hypoxia is known to block synaptic transmission of glutamatergic inputs⁸, GABAergic input^{8, 10} and cholinergic inputs^{11, 12}, causing disconnection, or synaptic 'arrest', of CA1 neuronal network⁸. These inputs and their interaction are known to play an essential role in enhancing synaptic efficacy in learning and memory¹³. Effects of brief hypoxia on synaptic transmission and of agents on hypoxic responses were monitored on responses of CA1 pyramidal cells to Sch activation. Brief hypoxia eliminated the EPSPs and EPSCs briefly (Fig. 2a,b; by 95.2±5.6%, n=10, and 96.8±4.2 %, respectively, *p*<0.05)g. The synaptic 'arrest' immediately disappeared when reoxygenation was initiated (Fig. 2a,b) and was not produced postsynaptically. Local application of glutamate during the last few seconds of the 3 min hypoxia revealed a peak inward current (201.2±10.5 pA) that differed insignificantly (n=7, *p*>0.05) from their control value (206.8±9.7 pA).

The hypoxic synaptic 'arrest' and reduction in cholinergic θ induction were prevented by blocking the adenosine A₁ receptors. Application of citicoline, a neuroprotective substance¹⁴, on the other hand, is ineffective (Fig. 3a; n=6,.*p*<0.05), suggesting that cellular injury was not involved. In the presence of 8-cyclopentyl-1,3-dipropylxanthine (CPDPX), a selective adenosine A₁ receptor antagonist, the synaptic transmission remained intact at the end of the hypoxia (Fig. 3b, 99.2±2.4% at the end of hypoxia versus control 100%; n=7,.*p*>0.05). Neither was θ activity (100.2±3.2%, n=6,.p>0.05) nor intracellular θ (99.6±3.0%, n=8,.*p*>0.05) affected by the brief hypoxia (Fig. 3c,d).

One of the most persistent consequences of transient hypoxia/ischemia is amnesia. Effects of brief hypoxia and CPDPX on spatial learning (Fig. 4a) were evaluated in rats, using a hidden-platform water maze. The episodes of brief hypoxia did not cause any obvious cell loss (Fig. 3e,f). As shown in Figure 4b, the latency to escape to the platform in all three groups of rats decreased following the training sessions. However, the group difference was significant (F_{2,27}=9.142,.*p*<0.001), indicating that spatial learning in rats subjected to brief hypoxia (hypoxia rats) was slower. A *post hoc* analysis reveals a significant difference from the 3rd trials (*p*<0.05). Quadrant tests 24 hrs after the last training trial revealed that the hypoxia rats (Fig. 4d) did not exhibit a quadrant preference (F_{3,36}=1.8,.*p*>0.05), whereas the control (F_{3,36}=160.3,.*p*<0.0001; Fig. 4c)) spent more time searching in the target quadrant (Quadrant 4) where the platform was previously placed. Thus, hypoxia rats performed worse than their controls in this spatial memory retention task.

The brief hypoxia-induced memory deficits were sensitive to CPDPX. Bilateral intracerebroventricular injections of CPDPX eliminated hypoxic impairment on the spatial memory (Fig. 4b). Quadrant tests revealed that CPDPX-hypoxia rats showed a preference for the target quadrant (F_{3,36}=169.7,.*p*<0.0001; Fig. 4e), identical to that of the control. The total swimming distances, however, did not differ between the three groups (Fig. 4f;.*p*>0.05).

Factors other than the extent of CA1 cell loss are also known to contribute to behavioral impairments^{15, 16}. Transient hypoxia/ischemia induces release of adenosine^{17, 19}, resulting in opening of both K*_{ATP}* and K_{Cn}²⁺ channels²⁰ and decreasing stimulus induced calcium influx into neurons²¹ via an action at presynaptic and postsynaptic A1 receptors. The reduction in cholinergic θ suggests an impaired temporal interaction of heterosynaptic inputs. For stable θ activity, some levels of ongoing activity and interaction of heterosynaptic inputs may be necessary. In addition, adenosine A₁ receptors are linked to G-proteins and perhaps via these facilitate the opening of potassium channels. Internal Ca²⁺ release from an InsP₃-sensitive internal store might be involved as a major component of the hypoxic response²². CA1 functional interference may underlie the observed spatial memory deficits due to brief hypoxia. The slightly enhanced EPSPs and LTP themselves, on the other hand, are unlikely to cause decreased spatial learning. Spatial learning has been reported to be normal with enhanced CA1 long-term potentiation by twofold in inositol 1,4,5-triphosphate 3-kinase_{A}-deficient mice²³. The episodes of brief hypoxia may be more relevant to a gradual memory decline during aging or Alzheimer's disease. A brief episode of global ischemia was reported to be sufficient to increase the production of amyloid precursor protein in vulnerable CA1 neurons²⁴. The hypoxic 'synaptic arrest' compromises the brain's ability to learn and memorize, which is an unnecessary sacrifice if the hypoxia turns out to be brief. Relieving the network from heterosynaptic 'arrest' through blocking the adenosine A₁ receptors may represent an effective strategy to eliminate the functional impairment. Combined with agents that reverse cellular injury and prevent cell loss, the antagonists might also be valuable in therapy against severe hypoxia/ischemia-induced memory loss.

### REFERENCES

The following citations are incorporated herein by reference:
1. Grubb, N.R., O'Carroll, R., Cobbe, S.M., Sirel, J. & Fox, K.A. Chronic memory impairment after cardiac arrest outside hospital. Br. Med. J. 313, 143-146 (1996).
2. Semenza, G.L. Perspectives on oxygen sensing Cell 98, 281-284 (1999).
3. Eu, J.P., Sun, J.H., Xu, I., Stamler, J.S. & Meissner, G. The skeletal muscle calcium release channel: coupled O2 sensor and NO signaling functions. Cell 102, 499-509 (2000).
4. Nakajimna, W., et al. Apoptosis has a prolonged role in the neurodegeneration after hypoxic ischemia in the newborn rat. J. Neurosci. 20, 7994-8004 (2000).
5. Sun, M.-K., Zhao, W.Q., Nelson, T.J. & Alkon, D.L. Theta rhythm of hippocampal CA1 neuron activity: gating by GABAergic synaptic depolarization. J. Neurophysiol in press, 2001.
6. Descarries, L., Gisiger, V. & Steriade, M. Diffuse transmission by acetylcholine in the CNS. Prog. Neurobiol. 53, 603-625 (1997).
7. Huerta, P.T. & Lisman, J.E. Bidirectional synaptic plasticity induced by a single burst during cholinergic theta oscillation in CA1 in vitro. Neuron 15, 1053-1063 (1995).
8. Hammond, C., Crépel, V., Gozlan, H. & Ben-Ari, Y. Anoxic LTD sheds light on the multiple facets of NMDA receptors. Trends Neurosci. 17, 497-503 (1994).
9. Arai, A., Larson, J. & Lynch, G. Anoxia reveals a vulnerable period in the development of long-term potentiation. Brain Res. 511, 353-357 (1990).
10. Congar, P, Khazipov, C.P. & Ben-Ari, Y. Direct demonstration of functional disconnection by anoxia of inhibitory interneurons form excitatory inputs in rat hippocampus. J. Neurophysiol. 73, 421-426 (1995).
11. Kasa, P., Rakonczay, Z. & Gulya, K. The cholinergic system in Alzheimer's disease. Prog. Neurobiol. 52, 511-535 (1997).
12. Porkka-Heiskanen, T., et al. Adenosine: A mediator of the sleep-inducing effects of prolonged wakefulness. Science 276, 1265-1267 (1997).
13. Shulz, D.E., Sosnik, R, Haidarliu, S. & Ahissar, E. A neuronal analogue of state-dependent learning. Nature 403, 549-553 (2000).
14. Shuaib, A., Yang, Y. & Li, Q. Evaluating the efficacy of citicoline in embolic ischemic stroke in rats: neuroprotective effects when used alone or in combination with urokinase. Exp. Neurol. 161, 733-739 (2000).
15. Gibson, G.E. & Duffy, T.E. Impaired synthesis of acetylcholine by mild hypoxic hypoxia or nitric oxide. J. Neurochem. 36, 28-33 (1981).
16. Jaspers, R.M.A., Block, F., Heim, C. & Sontag, K.-H. Spatial learning is affected by transient occlusion of common carotid arteris (2VO): comparison of behavioral and histopathological changes after '2VO' and 'four-vessel-occlusion' in rats. Neurosci. Lett. 117, 149-153 (1990).
17. Van Wylen, D.G., Park, T.S., Rubio, R. & Berne, R.M. Increases in cerebral interstitial fluid adenosine concentration during hypoxia, local potassium infusion, and ischemia. J. Cereb. Blood Flow Metab. 6, 522-528 (1986).
18. Sun, M.-K. Pharmacology of reticulospinal vasomotor neurons in cardiovascular regulation. Pharmacol. Rev. 48, 465-494 (1996).
19. Sun, M.-K. & Reis, D.J. Hypoxia-activated Ca2+ currents in pacemaker neuroes of rat rostral ventrolateral medulla in vitro. J. Physiol. Lond. 476, 101-116 (1994).
20. Yamamoto, S., Tanaka, E. & Higashi, H. Mediation by intracellular calcium-dependent signals of hypoxic hyperpolarization in rat hippocampal CA1 neurons in vitro. J. Neurophysiol. 77, 386-392 (1997).
21. Fredholm, B.B. & Dunwiddie, T.V. How does adenosine inhibit transmitter release? Trends Pharmacol, Sci. 9, 130-133 (1988).
22. Belousov, A.B., Godfraind, J.M. & Krnjevic, K. Internal Ca2+ stores involved in anoxic responses of rat hippocampal neurons. J. Physiol. Lond. 486, 547-556 (1995).
23. Jun, K., et al. Enhanced hippocampal CA1 LTP but not spatial learning in inositol 1,4,5-triphosphate 3-kinase(A)-deficient mice. Leanring Memory 5, 317-330 (1998).
24. Gervais, F.G., et al. Involvement of caspases in proteolytic cleavage of Alzheimer's amyloid-β precursor protein and amyloidogenic Aβ peptide formation. Cell 97, 395-406 (1999).
25. Furling, D., Ghribi, O., Lahsaini, A., Mirault, M.-E. & Massicotte, G. Impairment of synaptic transmission by transient hypoxia in hippocampal slices: improved recovery in glutathione peroxidase transgenic mice. Proc. Natl. Acad. Sci. USA 97, 4351-4356 (2000).

## Claims

1. The use of an adenosine A1 receptor antagonist for the manufacture of a pharmaceutical composition for the treatment of hypoxia-induced learning and/or memory impairment.

2. The use of an adenosine A1 receptor antagonist according to claim 1, wherein the hypoxia is cerebral.

3. The use of an adenosine A1 receptor antagonist according to any of the claims, wherein the hypoxia is reversible and transient.

4. The use of an adenosine A1 receptor antagonist according to any of the claims, wherein the hypoxia is induced by Alzheimer's disease, Parkinson's, Pugilistia, dementia or multiple sclerosis.

5. The use of an adenosine A1 receptor antagonist according to any of the claims, wherein the hypoxia occurs in repeated periods.

6. The use of an adenosine A1 receptor antagonist according to any of the claims, wherein the hypoxia is induced by traumatic events, transient ischemic attack, sugary-related hypoxia, acute and/or chronic obstructive lung disease, central nervous system infection such as meningitis encephalitis and/or other traumatic injury of the central nervous system.

7. The use of an adenosine A1 receptor antagonist according to any of the claims, wherein the antagonist is 8-cyclopentyl-1, 3-dipropylxanthine (CPDPX).

8. The use of an adenosine A1 receptor antagonist according to any of the claims, wherein the hypoxia reduces theta activity by least about 50% to about 95%.

9. The use of an adenosine A1 receptor antagonist according to any of the claims, wherein the antagonist mitigates the effects of hypoxia by restoring at least 75% to about 100% of pre-hypoxia levels for synaptic transmission and/or theta intracellular theta activity.

10. The use of an adenosine A1 receptor antagonist according to any of the claims, wherein the adenosine A 1 antagonist is combined with a pharmaceutically acceptable carrier, which delivers the antagonist across the blood brain barrier.

11. The use of an adenosine A1 receptor antagonist according to any of the claims, wherein the adenosine A 1 antagonist is combined with an agent that reverses cellular injury and/or prevents cell loss.

12. The use of an adenosine A1 receptor antagonist according to any of the claims, wherein the pharmaceutical composition comprises buffer solutions.

13. the use of an adenosine A1 receptor antagonist according to claim 12, wherein the buffer solution is a saline solution.

14. The use of an adenosine A1 receptor antagonist according to any of the claims, wherein the pharmaceutical composition can be formulated into solid, semi-solid, liquid, or gaseous preparations.

15. The use of an adenosine A1 receptor antagonist according to claim 14, wherein the pharmaceutical composition can be formulated into tablets, capsules, powders, granules, ointments, solutions, suppositories, injectables, inhalants and aerosols.

16. A method of identify a therapeutic A1 antagonist compound useful for treating hypoxia-related memory loss comprising:
providing brain tissue under controlled conditions modelling theta activity,
placing the tissue under conditions of hypoxia causing synaptic arrest and loss of theta activity,
administrating a candidate A-1 antagonist compound to brain tissue under conditions of hypoxia, and
determining whether the candidate compound prevents synaptic arrest of the brain tissue and/or maintains theta activity.

17. The method according to claim 16, wherein the brain tissue comprises CA1 pyramidal cells.

## Patentansprüche

1. Verwendung eines Adenosin-A1-Rezeptorantagonisten für die Herstellung einer pharmazeutischen Zusammensetzung für die Behandlung einer hypoxieinduzierten Lern- und/oder Gedächtnisschwäche.

2. Verwendung eines Adenosin-A1-Rezeptorantagonisten nach Anspruch 1, worin die Hypoxie zerebral ist.

3. Verwendung eines Adenosin-A1-Rezeptorantagonisten nach einem der Ansprüche, worin die Hypoxie reversibel und transient ist.

4. Verwendung eines Adenosin-A1-Rezeptorantagonisten nach einem der Ansprüche, worin die Hypoxie durch die Alzheimer-Krankheit, Parkinson'sche Krankheit, Pugilistica, Demenz oder multiple Sklerose induziert ist.

5. Verwendung eines Adenosin-A1-Rezeptorantagonisten nach einem der Ansprüche, worin die Hypoxie in wiederholten Perioden auftritt.

6. Verwendung eines Adenosin-A1-Rezeptorantagonisten nach einem der Ansprüche, worin die Hypoxie durch traumatische Ereignisse, transitorische ischämische Attacken, eine zuckerbedingte Hypoxie, eine akute und/oder chronische obstruktive Lungenerkrankung, eine Infektion des Zentralnervensystems wie eine Meningitis, Enzephalitis und/oder eine andere traumatische Verletzung des Zentralnervensystems induziert ist.

7. Verwendung eines Adenosin-A1-Rezeptorantagonisten nach einem der Ansprüche, worin der Antagonist 8-Cyclopentyl-1,3-Dipropylxanthin (CPDPX) ist.

8. Verwendung eines Adenosin-A1-Rezeptorantagonisten nach einem der Ansprüche, worin die Hypoxie die Theta-Aktivität um mindestens etwa 50 % bis 95 % verringert.

9. Verwendung eines Adenosin-A1-Rezeptorantagonisten nach einem der Ansprüche, worin der Antagonist die Effekte der Hypoxie lindert, indem er mindestens 75 % bis etwa 100 % der Prähypoxielevel für die synaptische Transmission und/oder die intrazelluläre Theta-Aktivität wiederherstellt.

10. Verwendung eines Adenosin-A1-Rezeptorantagonisten nach einem der Ansprüche, worin der Adenosin-A1-Antagonist mit einem pharmazeutisch akzeptablen Träger kombiniert wird, der den Antagonisten über die Blut-Hirn-Schranke bringt.

11. Verwendung eines Adenosin-A1-Rezeptorantagonisten nach einem der Ansprüche, worin der Adenosin-A1-Antagonist mit einem Mittel kombiniert ist, das die zelluläre Verletzung umkehrt und/oder allen Zellverlust verhindert.

12. Verwendung eines Adenosin-A1-Rezeptorantagonisten nach einem der Ansprüche, worin die pharmazeutische Zusammensetzung Pufferlösungen umfasst.

13. Verwendung eines Adenosin-A1-Rezeptorantagonisten nach Anspruch 12, worin die Pufferlösung eine Salzlösung ist.

14. Verwendung eines Adenosin-A1-Rezeptorantagonisten nach einem der Ansprüche, worin die pharmazeutische Zusammensetzung in festen, halbfesten, flüssigen oder gasförmigen Präparaten formuliert werden kann.

15. Verwendung eines Adenosin-A1-Rezeptorantagonisten nach Anspruch 14, worin die pharmazeutische Zusammensetzung in Tabletten, Kapseln, Pulvern, Ganula, Salben, Lösungen, Suppositorien, Einspritzmitteln, Inhalationsmitteln und Aerosolen formuliert werden kann.

16. Verfahren zur Identifizierung einer therapeutischen A1-Antagonistenverbindung, die für die Behandlung eines hypoxiebedingten Gedächtnisverlusts nützlich ist, umfassend:
Bereitstellung von Hirngewebe und kontrollierten Bedingungen, die die Theta-Aktivität modellieren,
Plazieren des Gewebes unter Hypoxiebedingungen, die einen synaptischen Arrest und einen Verlust der Theta-Aktivität verursachen,
Verabreichung einer Kandidaten-A1-Antagonistenverbindung an das Hirngewebe und Hypoxiebedingungen, und
Bestimmen, ob die Kandidatenverbindung den synaptischen Arrest des Hirngewebes verhindert und/oder die Theta-Aktivität aufrechterhält.

17. Verfahren nach Anspruch 16, worin das Hirngewebe CA1 Pyramidalzellen umfasst.

## Revendications

1. Utilisation d'un antagoniste du récepteur de l'adénosine A1 pour la fabrication d'une composition pharmaceutique pour le traitement de troubles de l'apprentissage et/ou de la mémoire induits par une hypoxie.

2. Utilisation d'un antagoniste du récepteur de l'adénosine A1 selon la revendication 1, dans laquelle l'hypoxie est cérébrale.

3. Utilisation d'un antagoniste du récepteur de l'adénosine A1 selon l'une quelconque des revendications, dans laquelle l'hypoxie est réversible et transitoire.

4. Utilisation d'un antagoniste du récepteur de l'adénosine A1 selon l'une quelconque des revendications, dans laquelle l'hypoxie est induite par la maladie d'Alzheimer, la maladie de Parkinson, la dementia pugilistica, la démence, ou la sclérose en plaque.

5. Utilisation d'un antagoniste du récepteur de l'adénosine A1 selon l'une quelconque des revendications, dans laquelle l'hypoxie survient de manière répétée.

6. Utilisation d'un antagoniste du récepteur de l'adénosine A1 selon l'une quelconque des revendications, dans laquelle l'hypoxie est induite par des événements traumatiques, un AVC ischémique transitoire, une hypoxie liée aux sucres, une maladie pulmonaire obstructive aiguë et/ou chronique, une infection du système nerveux central comme une méningite, une encéphalite et/ou une autre lésion traumatique du système nerveux central.

7. Utilisation d'un antagoniste du récepteur de l'adénosine A1 selon l'une quelconque des revendications, dans laquelle l'antagoniste est la 8-cyclopentyl-1, 3-dipropylxanthine (CPDPX).

8. Utilisation d'un antagoniste du récepteur de l'adénosine A1 selon l'une quelconque des revendications, dans laquelle l'hypoxie réduit l'activité thêta d'au moins environ 50 % à environ 95 %.

9. Utilisation d'un antagoniste du récepteur de l'adénosine A1 selon l'une quelconque des revendications, dans laquelle l'antagoniste atténue les effets de l'hypoxie en restaurant au moins 75 % à environ 100 % des niveaux pré-hypoxiques de transmission synaptique et/ou d'activité intracellulaire thêta.

10. Utilisation d'un antagoniste du récepteur de l'adénosine A1 selon l'une quelconque des revendications, dans laquelle l'antagoniste de l'adénosine A1 est combiné à un véhicule pharmaceutiquement acceptable qui administre l'antagoniste à travers la barrière hémato-encéphalique.

11. Utilisation d'un antagoniste du récepteur de l'adénosine A1 selon l'une quelconque des revendications, dans laquelle l'antagoniste de l'adénosine A1 est combiné à un agent qui traite les lésions cellulaires et/ou empêche la perte de cellules.

12. Utilisation d'un antagoniste du récepteur de l'adénosine A1 selon l'une quelconque des revendications, dans laquelle la composition pharmaceutique comprend des solutions tampon.

13. Utilisation d'un antagoniste du récepteur de l'adénosine A1 selon la revendication 12, dans laquelle la solution tampon est une solution saline.

14. Utilisation d'un antagoniste du récepteur de l'adénosine A1 selon l'une quelconque des revendications, dans laquelle la composition pharmaceutique peut être formulée en préparations solides, semi-solides, liquides ou gazeuses.

15. Utilisation d'un antagoniste du récepteur de l'adénosine A1 selon la revendication 14, dans laquelle la composition pharmaceutique peut être formulée en comprimés, capsules, poudres, granules, onguents, solutions, suppositoires, produits injectables, produits pour inhalation et aérosols.

16. Procédé pour identifier un composé thérapeutique antagoniste de A1 utile pour traiter la perte de mémoire liée à l'hypoxie, comprenant les étapes consistant à :
fournir du tissu cérébral dans des conditions contrôlées modelant l'activité thêta,
placer le tissu dans des conditions d'hypoxie provoquant un arrêt synaptique et une perte d'activité thêta,
administrer un composé candidat antagoniste de A1 au tissu cérébral dans des conditions d'hypoxie, et
déterminer si le composé candidat empêche l'arrêt synaptique du tissu cérébral et/ou maintient l'activité thêta.

17. Procédé selon la revendication 16, dans lequel le tissu cérébral comprend des cellules pyramidales CA1.
